# EUROPEAN PATENT SPECIFICATION

(11) **EP 1 050 529 B1**
(45) Date of publication and mention of the grant of the patent: **22.10.2003**
(21) Application number: 00303586.2
(22) Date of filing: 28.04.2000
(51) Int. Cl.: C07C 255/24, C08K 5/16, A01N 37/34

(54) **Aminobutyronitrile compositions**
Aminobutyronitril Mischung
Mélange d'aminobutyronitrile

(30) Priority: 03.05.1999 US 303850; 03.05.1999 US 304401
(43) Date of publication of application: 08.11.2000
(73) Proprietor: BASF AKTIENGESELLSCHAFT, 67056 Ludwigshafen (DE)
(72) Inventor: Gastrock, William Henry, West Helena, Arizona 72390 (US); Wepplo, Peter John, Princeton, New Jersey 08540 (US); Kremer, Kenneth Alfred Martin, Lawrencefille, New Jersey 08648 (US); Drabb, Thomas Walter, Trenton, New Jersey 08611 (US)
(74) Representative: Langfinger, Klaus Dieter, Dr.

(56) References cited:
- EP-A- 0 123 830
- US-A- 4 683 324

## Description

Phenoxypropionic acid cyanimide derivatives, such as those described in EP 262,393 and Research Disclosure 92306005, are useful as fungicides, particularly for the control of the causative agents of rice blast. Said cyanimide derivatives contain assymetric or stereogenic carbon atoms and it has been demonstrated that those derivatives having the R-configuration show enhanced fungicidal activity over that of the corresponding racemic mixtures. Similarly, the imidazolinone family of herbicides, such as those described in U.S. 4,798,619 and U.S. 5,334,576, contain assymetric or stereogenic carbon atoms and it has been demonstrated that those imidazolinones having the R-configuration on the dialkylsubstituted carbon atom in the imidazolinone ring show a greater herbicidal activity than the corresponding racemic mixtures.

A common key chiral intermediate compound, (R)2-amino-2,3-butyronitrile may be used to prepare the above-said agriculturally active compounds. However, said (R)aminobutyronitrile compound is unstable and readily racemizes upon standing, thus making practical manufacturing procedures difficult.

Therefore, it is an object of this invention to provide a stable (R)2-amino-2,3-dimethylbutyronitrile composition useful for the manufacture of agriculturally active compounds.

It is another object of this invention to provide a readily available, storage-stable source of (R)2-amino-2,3-dimethylbutyronitrile.

### SUMMARY OF THE INVENTION

The present invention provides a stable chiral composition which comprises 15 to 40% by weight of (R)2-amino-2,3-dimethylbutyronitrile and a substantially water-free non-polar solvent. Said compositions are useful as intermediates in the manufacture of agriculturally active agents such as fungicidal cyanimides and herbicidal imidazolinones having the R-configuration.

### DETAILED DESCRIPTION OF THE INVENTION

Fungicidal α-phenoxypropionic acid cyanimide derivatives and their preparation from (R)2-amino-2,3-dimethylbutyronitrile are described in Research Disclosure 92306005. Herbicidal imidazolinones and their preparation from (R)2-amino-2,3-dimethylbutyronitrile are described in U.S. 4,683,324. Said patent also describes the preparation and isolation of (R)2-amino-2,3-dimethylbutyronitrile. Although said (R)aminobutyronitrile may be potentially useful as a key common intermediate in the manufacture of agriculturally active agents such as fungicides and herbicides, its half-life is estimated to be less than 8 hours at room temperature, therefore, making its use in a manufacturing procedure highly impractical.

Surprisingly, it has now been found that a composition which comprises 15% to 40%, by weight of (R)2-amino-2,3-dimethylbutyronitrile (hereinafter designated R-aminonitrile) and a substantially water-free non-polar solvent is storage-stable for prolonged periods of time at temperatures at or below room temperature (up to about 25°C). Higher temperatures or higher concentrations may be employed, in the inventive compositions, however higher temperatures or higher concentrations accelerate the racemization process while lower temperatures or lower concentrations decrease the rate of racemization and increase the storage-stable period of time.

Advantageously, the composition of the invention may be employed in a practical manufacturing procedure, such as a process to prepare fungicidal α-phenoxycyanimides or herbicidal imidazolinones having the R configuration, without rapid decomposition due to racemization of, or loss of HCN from, the R-aminonitrile starting material. Further, the stability of the composition of the invention allows for interim storage or transportation of the R-aminonitrile compound as needed for manufacturing purposes. It is intended that the stable chiral aminobutyronitrile compositions of the invention also embrace the corresponding essentially enantiomerically pure (S)2-amino-2,3-dimethylbutyronitrile compound as the chiral component therein.

Non-polar solvents useful in the composition of the invention are aromatic hydrocarbons (e.g. toluene, benzene, xylene, naphthalene and the like preferably toluene), halogenated aromatic hydrocarbons (e.g. chlorobenzene, dichlorobenzenes and the like), hydrocarbons (e.g. pentanes, hexanes and the like), halogenated hydrocarbons (e.g. chloroform, methylene chloride, dichloroethane, and the like, esters (e.g. ethyl acetate, methyl propionate and the like), ethers (e.g. diethyl ether, tetrahydrofuran, dioxane and the like) or any of the conventional, preferably water immiscible, organic non-polar solvents.

Preferred non-polar solvents suitable for the composition of the invention are aromatic hydrocarbons, particularly toluene.

In order to facilitate a further understanding of the invention, the following examples are presented primarily for the purpose of illustrating certain more specific details thereof. The invention is not to be deemed limited thereby except as defined in the claims.

Unless otherwise noted, all parts are parts by weight. HPLC designates high performance liquid chromatography.

### EXAMPLE 1

### Evaluation Of The Solvent Effect On The Racemization Of A 10% Solution of (R)2-Amino-2,3-dimethylbutyronitrile

### A) Preparation of (R)2-Amino-2,3-dimethylbutyronitrile

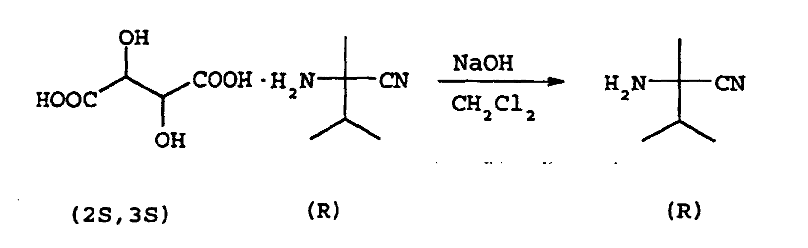

A mixture of methylene chloride, ice, (R)2-amino-2,3-dimethylbutyronitrile (2S,3S) tartaric acid salt (8.13g, 31.0 mmol) and 50% NaOH (5.3 ml, 8.0 g, 100 mmol NaOH) is shaken until no solid particles are observed. The organic phase is separated, dried over MgSO₄ and filtered. The filtrate is distilled *in vacuo* at 20°C to remove the methylene chloride and obtain free (R)2-amino-2,3-dimethylbutyronirile as a clear liquid, 3.42 g (98.3% yield).

### B) Optical Rotation Evaluation

In these evaluations, 10% wt/wt solutions of the freshly prepared (R)2-amino-2,3-dimethylbutyronitrile in a variety of solvents are placed in a constant temperature bath. Optical rotations, ([α]_{D}) are determined at time 0 and at regular intervals thereafter. The data obtained are shown in Tables I and II.

**Table I**

| **Evaluation of Non-polar Solvent Effect On Stability Of (R)2-Amino,2,3-dimethylbutronitrile Compositions** | | | | |
|---|---|---|---|---|
| **Solvent** | **Time (Hr.)** | **[α]**_{**D**} | **Δ**^{**1**}**[α]**_{**D**} | **Temperature (°C)** |
| | | | | |
| Ethyl Acetate | 0 | -00.422 | | 26 |
| | 1 | -00.422 | | 26 |
| | 2.5 | -00.425 | | 26 |
| | 3.5 | -00.424 | -0.002 | 26 |
| | 4.5 | -00.424 | | 26 |
| | 19.5 | -00.421 | | 26 |
| | | -00.423 | | 26 |
| | | | | |
| Toluene | 0 | -00.423 | | 26 |
| | 1 | -00.423 | | 26 |
| | 2 | -00.423 | | 26 |
| | 7 | -00.423 | 0.000 | 26 |
| | 23 | -00.423 | | 26 |
| | | | | |
| Acetonitrile | 0 | -00.209 | | 26 |
| | 1 | -00.204 | | 26 |
| | 2 | -00.199 | | 26 |
| | 3 | -00.197 | 0.012 | 26 |
| | 312 | -00.025 | | 26 |
| Tetrahydrofuran | 0 | -00.520 | | 26 |
| | 1 | -00.520 | | 26 |
| | 2 | -00.518 | | 26 |
| | 4.5 | -00.518 | 0.002 | 26 |
| | 27 | -00.515 | | 26 |
| | | | | |
| Methylene Chloride | 0 | -00.468 | | 26 |
| | 1 | -00.467 | | 26 |
| | 2 | -00.461 | 0.007 | 26 |
| | 17.5 | -00.458 | | 26 |
| | | | | |
| Chloroform | 0 | -00.547 | | 26 |
| | 1 | -00.549 | | 26 |
| | 2 | -00.547 | 0.000 | 26 |
| | 17.5 | -00.540 | | 26 |
| | | | | |
| Dimethyl Formamide | 0 | -00.081 | | 26 |
| | 1 | -00.074 | | 26 |
| | 3 | -00.062 | 0.019 | 26 |
| | 23 | -00.015 | | 26 |
| | 168 | +00.002 | | 26 |
| | | | | |
| Ethyl Ether | 0 | -00.505 | | 26 |
| | 1 | -00.505 | | 26 |
| | 4 | -00.502 | 0.003 | 26 |
| | 6 | -00.504 | | 26 |
| | | | | |
| Hexanes | 0 | -00.492 | | 26 |
| | 1 | -00.494 | | 26 |
| | 4 | -00.491 | 0.001 | 26 |
| | 6 | -00.482 | | 26 |
| | | | | |
| Chlorobenzene | 0 | -00.306 | | 25 |
| | 1 | -00.304 | | 25 |
| | 3 | -00.289 | 0.017 | 25 |
| | 20.5 | -00.285 | | 25 |
| | | | | |
| o-Dichlorobenzene | 0 | -00.242 | | 25 |
| | 1 | -00.241 | | 25 |
| | 3 | -00.240 | 0.002 | 25 |
| | 20.5 | -00.229 | | 25 |
| | | | | |
| Nitrobenzene | 0 | -00.068 | | 25 |
| | 1 | -00.051 | | 25 |
| | 3 | -00.045 | 0.023 | 25 |
| | 20.5 | -00.053 | | 25 |
| | | | | |
| 1,2-Dichloroethane | 0 | -00.419 | | 25 |
| | 2 | -00.408 | | 25 |
| | 4 | -00.421 | -0.002 | 25 |
| | 6 | -00.436 | | 25 |
| | | | | |
| 1,2-Dimethoxyethane | 0 | -00.493 | | 25 |
| | 2 | -00.494 | | 25 |
| | 4 | -00.508 | -0.015 | 25 |
| | 6 | -00.514 | | 25 |
| | | | | |
| 2-Butanone | 0 | -00.368 | | 25 |
| | 2 | -00.366 | | 25 |
| | 4 | -00.358 | 0.014 | 25 |
| | 6 | -00.357 | | 25 |
| | | | | |
| Xylenes | 0 | -00.447 | | 25 |
| | 2 | -00.442 | | 25 |
| | 4 | -00.453 | -0.006 | 25 |
| | 6 | -00.460 | | 25 |

| | | | | |
|---|---|---|---|---|
| ¹Δ [α]_{D} = [α]_{D} at Time 0 minus [α]_{D} at Time T | | | | |

**Table II**

| **Comparative Evaluation of Polar Solvent Effect On Stability Of (R)2-Amino-2,3-dimethylbutronitrile Compositions** | | | | |
|---|---|---|---|---|
| **Solvent** | **Time (Hr.)** | **[α]**_{**D**} | **Δ**^{**1**} **[α]**_{**D**} | **Temperature (°C)** |
| | | | | |
| Methanol | 0 | -00.500 | | 26 |
| | 1 | -00.173 | | 27 |
| | 2 | -00.054 | | 27 |
| | 3 | -00.015 | 0.485 | 26 |
| | | | | |
| (±)2-Butanol | 0 | -00.541 | | 26 |
| | 1 | -00.425 | | 26 |
| | 2 | -00.359 | | 26 |
| | 4.5 | -00.230 | 0.311 | 26 |
| | 27 | -00.000 | | 26 |
| | | | | |
| Dimethylsulfoxide | 0 | -00.239 | | 26 |
| | 1 | -00.151 | | 26 |
| | 3 | -00.059 | 0.180 | 26 |
| | 23 | +00.003 | | 26 |
| | 168 | -00.003 | | 26 |
| | | | | |
| Ethanol | 0 | -00.540 | | 26 |
| | 1 | -00.298 | | 26 |
| | 2 | -00.145 | | 26 |
| | 4 | -00.064 | 0.476 | 26 |
| | 6 | -00.022 | | |

| | | | | |
|---|---|---|---|---|
| ¹Δ [α]_{D} = [α]_{D} at Time 0 minus [α]_{D} at Time T | | | | |

As can be seen from the data shown in Tables I and II above, racemization is decreased by a factor of 10 to 100 fold when the chiral compound is present as a 10% solution in a non-polar solvent as compared to when it is present as a 10% solution in a polar solvent.

### EXAMPLE 2

### Comparative Evaluation Of The Effect Of Water On The Racemization Of A Toluene Solution Of (R)2-Amino-2,3-dimethylbutyronitrile

In this evaluation, (R)2(R)2-amino-2,3-dimethylbutyronitrile is prepared in a manner similar to that described in part A of Example 1 and employing toluene in place of methylene chloride. Upon extraction and separation, a 25.8% solution of free 2-amino-2,3-dimethylbutyronitrile in toluene is obtained. Immediately after extraction, the % R isomer of the water wet toluene solution is determined by HPLC analysis. The wet solution is stored at 25°C for 24 hours and a second measurement is taken. The wet solution is then dried azeotropically (45°-50°C/60-65 mmHg), analyzed for % R isomer by chiral HPLC immediately after drying, stored at 25°C for 4 days and analyzed a second time. The results are shown in Table III.

**Table III**

| **Comparative Evaluation Of The Effect Of Water On The Stability Of (R)2-Amino,2,3-dimethylbutronitrile Compositions** | | | |
|---|---|---|---|
| **Solvent** | **Time (Days)** | **% R Isomer** | **Δ% R**^{**1**} |
| Wet Toluene (comparison) | 0 | 85.2 | |
| Wet Toluene (comparison | 1 | 80.3 | -4.9 |
| | | | |
| Dry Toluene (invention) | 0 | 79.5 | |
| Dry Toluene (invention) | 4 | 78.9 | -0.6 |

| | | | |
|---|---|---|---|
| ¹Δ%R = % R at Time 0 minus % R at Time T | | | |

As can be seen from the data in Table III above, solutions of the chiral compound in essentially the absence of water are significantly more stable than those solutions in which water is present.

### EXAMPLE 3

### Evaluation Of The Effect Of Temperature And Concentration On The Racemization Of A Solution of (R)2-Amino-2,3-dimethylbutyronitrile

In these evaluations, the test solution is prepared in essentially the same manner as described in Example 2 and the solution is azeotropically dried immediately following extraction. A 50 g sample of the thus-prepared test solution is introduced into a 3-necked round bottom flask which has been set at a predetermined temperature and flushed with nitrogen. Samples of the test solution are taken directly from the flask at 0, 4, 24 and 48 hour intervals and analyzed for % R isomer and wt % concentration of (R,S)2-amino-2,3-dimethylbutyronitrile by chiral HPLC. The data obtained are shown in Table IV.

**Table IV**

| **Evaluation of The Effect Of Concentration And Temperature On the Stability Of (R)2-Amino,2,3-dimethylbutyronitrile Compositions** | | | | |
|---|---|---|---|---|
| **Concentration (wt %)** | **Temperature (°C)** | **Time (Hr.)** | **%R Isomer** | **Δ% R**^{**1**} |
| 19.2 | 15 | 0 | 94.0 | |
| 19.5 | 15 | 4 | 93.9 | |
| 19.4 | 15 | 24 | 93.9 | |
| 19.2 | 15 | 48 | 93.6 | 0.4 |
| | | | | |
| 19.2 | 20 | 0 | 94.2 | |
| 19.2 | 20 | 24 | 94.0 | |
| 19.2 | 20 | 48 | 94.0 | 0.2 |
| | | | | |
| 19.2 | 60 | 0 | 94.0 | |
| 19.5 | 60 | 4 | 91.9 | |
| 19.8 | 60 | 24 | 81.6 | |
| 20.2 | 60 | 48 | 71.6 | 22.4 |
| | | | | |
| 32.2 | 20 | 0 | 93.5 | |
| 32.2 | 20 | 48 | 93.1 | 0.4 |
| | | | | |
| 38.6 | 2 | 0 | 93.4 | |
| 37.2 | 2 | 144 | 92.9 | 0.5 |
| 38.7 | 2 | 336 | 91.7 | |
| | | | | |
| 38.6 | 35 | 0 | 93.4 | |
| 39.0 | 35 | 24 | 86.9 | |
| 38.1 | 35 | 48 | 84.1 | 9.3 |
| | | | | |
| 38.3 | 20 | 0 | 90.5 | |
| 38.3 | 20 | 24 | 89.5 | |

| | | | | |
|---|---|---|---|---|
| ¹Δ%R = %R at Time 0 minus %R at 48 hr. | | | | |

**Table IV**

| **Concentration (wt %)** | **Temperature (°C)** | **Time (Hr.)** | **%R Isomer** | **Δ% R**^{**1**} |
|---|---|---|---|---|
| | | | | |
| 37.2 | 35 | 0 | 92.9 | |
| 36.8 | 35 | 4 | 91.4 | |
| 36.8 | 35 | 24 | 88.4 | |
| 39.2 | 35 | 48 | 84.3 | 8.6 |
| | | | | |
| 45.9 | 45 | 0 | 94.4 | |
| 46.5 | 47 | 2 | 89.4 | |
| | | | | |
| 65.2 | 15 | 0 | 92.8 | |
| 63.8 | 15 | 4 | 92.6 | |
| 64.9 | 15 | 24 | 90.8 | |
| 63.4 | 15 | 48 | 89.9 | 2.9 |
| | | | | |
| 65.2 | 60 | 0 | 92.8 | |
| 64.4 | 60 | 4 | 71.3 | |
| 63.4 | 60 | 24 | 51.4 | |
| 66.0 | 60 | 48 | 50.5 | 42.4 |

| | | | | |
|---|---|---|---|---|
| ¹Δ%R = %R at Time 0 minus %R at 48 hr. | | | | |

As can be seen from the data shown in Table IV above, high concentration combined with high temperature decreases the stability of the chiral solution, however concentrations as high as 65% may be stable at moderate temperature.

## Claims

1. A stable composition which comprises 15% to 40% by weight of (R)2-amino-2,3-dimethylbutyronitrile and a substantially water-free non-polar solvent.

2. The composition according to claim 1 wherein the solvent is selected from the group consisting of aromatic hydrocarbons, halogenated aromatic hydrocarbons, hydrocarbons, halogenated hydrocarbons, esters and ethers.

3. The composition according to claim 2 wherein the solvent is an aromatic hydrocarbon.

4. The composition according to claim 3 wherein the solvent is toluene.

## Patentansprüche

1. Stabile Zusammensetzung, die 15 Gew.-% bis 40 Gew.-% (R)2-Amino-2,3-dimethylbutyronitril und ein im wesentlichen wasserfreies unpolares Lösungsmittel enthält.

2. Zusammensetzung nach Anspruch 1, wobei das Lösungsmittel aus der Gruppe aromatische Kohlenwasserstoffe, halogenierte aromatische Kohlenwasserstoffe, Kohlenwasserstoffe, halogenierte Kohlenwasserstoffe, Ester und Ether stammt.

3. Zusammensetzung nach Anspruch 2, wobei es sich bei dem Lösungsmittel um einen aromatischen Kohlenwasserstoff handelt.

4. Zusammensetzung nach Anspruch 3, wobei es sich bei dem Lösungsmittel um Toluol handelt.

## Revendications

1. Composition stable comprenant entre 15 et 40% par poids de (R)2-amino-2,3-diméthylbutyronitrile et un solvant apolaire sensiblement anhydre.

2. Composition selon la revendication 1 dans laquelle le solvant est sélectionné parmi le groupe constitués d'hydrocarbures aromatiques, d'hydrocarbures aromatiques halogénés, d'hydrocarbures, d'hydrocarbures halogénés, d'esters et d'éthers.

3. Composition selon la revendication 2 dans laquelle le solvant est un hydrocarbure aromatique.

4. Composition selon la revendication 3 dans laquelle le solvant est le toluène.
